# EUROPÄISCHE PATENTSCHRIFT

(11) **EP 2 341 960 B1**
(45) Veröffentlichungstag und Bekanntmachung des Hinweises auf die Patenterteilung: **24.04.2019**
(21) Anmeldenummer: 09778024.1
(22) Anmeldetag: 21.08.2009
(51) Int. Cl.: A61M 5/30

(54) **NADELLOSER EINWEGINJEKTOR MIT HOHER INJEKTIONSSICHERHEIT**
NEEDLE-LESS, DISPOSABLE INJECTOR PROVIDED WITH HIGH INJECTION SAFETY
INJECTEUR SANS AIGUILLE À USAGE UNIQUE SE CARACTÉRISANT PAR UNE SÉCURITÉ D'INJECTION ÉLEVÉE

(30) Priorität: 23.09.2008 DE 102008048595
(43) Veröffentlichungstag der Anmeldung: 13.07.2011
(73) Patentinhaber: LTS LOHMANN Therapie-Systeme AG, 56626 Andernach (DE)
(72) Erfinder: ASMUSSEN, Bodo, 22949 Ammersbek (DE); HOFFMANN, Hans-Rainer, 56566 Neuwied (DE)
(74) Vertreter: Thämer, Wolfgang
(86) Internationale Anmeldenummer: PCT/EP2009/006065
(87) Internationale Veröffentlichungsnummer: WO 2010/034380

(56) Entgegenhaltungen:
- EP-A1- 1 336 419
- WO-A1-2005/044344
- WO-A1-2009/012855
- DE-B3-102005 062 206
- US-A1- 2002 099 329
- US-B1- 6 517 517

## Beschreibung

Die Erfindung betrifft einen nadellosen Einweginjektor mit einem Gehäuse, das mindestens einen Druckstab umfasst, mit einer Zylinder-Kolben-Einheit, mit einem einen Stempelteller aufweisenden Kolbenbetätigungsstempel, mit einer ein hülsenartiges Auslöseelement aufweisenden Auslöseeinheit und mit einem zwischen einem Boden des Gehäuses und dem Stempelteller vorgespannt sitzenden Federenergiespeicher, wobei beim gespannten Einweginjektor der Stempelteller über eine Bundfläche auf einer Abstützfläche des Druckstabs aufliegt, wobei eine Anlagefläche eines Nockens des Druckstabs eine Innenwandung des Auslöseelements kontaktiert, wobei das Auslöseelement eine scharfe Kante aufweist, wobei die Auslöseeinheit den vorgespannten Federenergiespeicher zunächst mittels des Druckstabs verriegelt, wobei das Auslöseelement in Richtung der Zylinder-Kolben-Einheit verschiebbar ist, wobei beim Auslösen der Druckstab am Auslöseelement entlang gleitet und die Anlagefläche über die Kante rutscht ist und wobei nach dem Auslösen der Auslöseeinheit der Federenergiespeicher mittels des Kolbenbetätigungsstempels den Druckstab verdrängt und die Zylinder-Kolben-Einheit betätigt.

Aus der nachveröffentlichten DE 10 2007 034 871 ist ein derartiger Einweginjektor bekannt. Wird dieser Injektor mit zu geringem Druck auf die Injektionsstelle ausgesetzt, besteht die Gefahr, dass Injektionslösung in seitlicher Richtung zur Achse des Injektionsstrahls entweicht. Es kommt zu einem sogenannten wet-shot.

Elemente eines derartigen Injektors sind auch aus der EP 1 336 419 A1 bekannt, Diese Druckschrift offenbart einen Strahlinjektor, umfassend ein Gehäuse, eine Fluidkammer, einen Kolben, eine Kraft erzeugende Quelle, ein Aktivierungselement zum Aktivieren der Kraft erzeugenden Quelle und einen Nadelschutz, welcher am distalen Ende des Gehäuses zum Verdecken einer Nadel angeordnet ist. Bei diesem Nadelinjektor werden die den Kolbenbetätigungsstempel haltenden Nocken des Innengehäuses von einer von der Zylinder-Kolben-Einheit weg mittels eines Nadelschutzes verschiebbaren, die Vorschubfeder abstützende Hülse in ihrer Position gehalten.

Die WO 2005 / 044344 A1 offenbart einen Nadelinjektor, dessen Einstechen gegen die Kraft einer Rückstellfeder für eine Nadelhülse erfolgt. Die Anpresskraft wird von der Federkraft der Rückstellfeder bestimmt.

Aus der US 2002 / 0099 329 A1 ist ein gasbetriebenes nadelloses Injektionsgerät bekannt, wobei durch axiales verschieben einer Kappe eine Kanüle in eine Gaspatrone gedrückt wird.

Aus der DE 10 2005 062 206 B3 ist ein Einweginjektor mit einem Gehäuse bekannt, in dem mindestens ein mechanischer Federenergiespeicher, mindestens eine - zumindest zeitweise wirkstoffbefüllbare - Zylinderkolbeneinheit, mindestens ein Kolbenbetätigungsstempel und mindestens eine Auslöseeinheit angeordnet ist. Dabei umfasst der Federenergiespeicher ein vorgespanntes Federelement, dass von einem dieses zumindest bereichsweise umgebenden Zugmittel in der vorgespannten Position gehalten wird. Weiterhin umfasst die Auslöseeinheit ein Schneidwerkzeug, das zur Freigabe der Energie des Federenergiespeichers das Zugmittel an mindestens einer Stelle durchtrennt oder schwächt, wobei die Schwächung ein sofortiges Reißen des Zugmittels bewirkt.

Der vorliegenden Erfindung liegt daher die Problemstellung zugrunde, einen Einweginjektor zu entwickeln, bei dessen Einsatz die erforderliche Mindestanpresskraft sichergestellt ist.

Diese Problemstellung wird mit den Merkmalen des Hauptanspruchs gelöst. Dazu ist die Innenwandung in dem Bereich, an dem die Anlagefläche entlang gleitet, zur Erhöhung des Gleitreibungskoeffizienten aufgeraut oder strukturiert. Bei Belastung durch den vorgespannten Federenergiespeicher weist die Paarung der einander zugewandten Innenwandung des Auslöseelements und der Anlagefläche des Druckstabs zumindest bereichsweise einen höheren Gleitreibungskoeffizienten auf als die Paarung der einander zugewandten Bundfläche des Kolbenbetätigungsstempels und der Abstützfläche des Druckstabs.

Weitere Einzelheiten der Erfindung ergeben sich aus den Unteransprüchen und den nachfolgenden Beschreibungen schematisch dargestellter Ausführungsbeispiele.

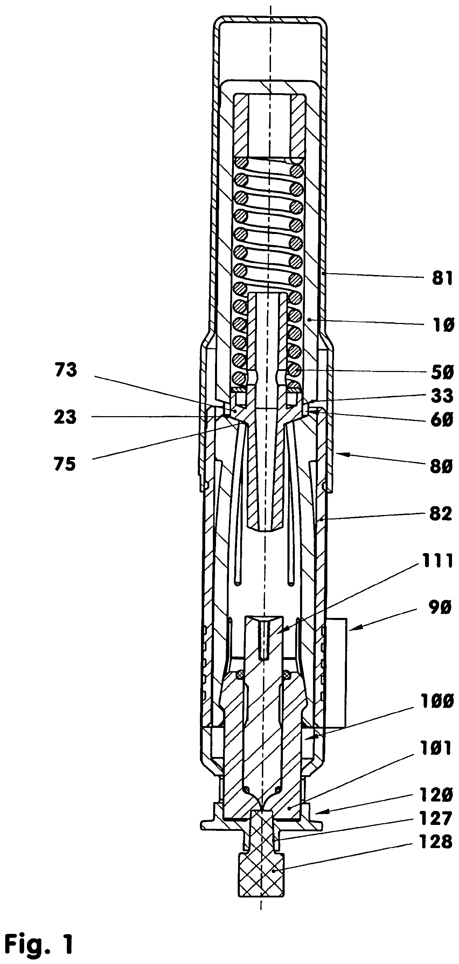

Der in den Figuren 1 - 3 dargestellte Einweginjektor besteht aus einem Gehäuse (10), einer Zylinder-Kolben-Einheit (100), einem Kolbenbetätigungsstempel (60) und einer Schraubendruckfeder (50) als Federenergiespeicher. Zudem sind am Gehäuse (10) eine Auslöseeinheit (80) mit einem Auslöseelement (82) und ein Sicherungselement (90) angeordnet. Die Zylinder-Kolben-Einheit (100) ist in der Darstellung der Figur 1 vorne mittels einer Verschlusskappe (120) in Kombination mit einem Stopfen (128) verschlossen.

Das Gehäuse (10) ist ein einteiliger, topfförmiger, unten offener Hohlkörper mit obenliegendem Boden (39). Es wird z.B. aus einem glasfaserverstärkten Polyamid durch Spritzgießen gefertigt. Das Gehäuse (10) hat eine weitgehend rohrförmige Gestalt und ist in zwei Funktionsbereiche aufgeteilt, das ist zum einen der obere Mantelbereich (31) und zum anderen der untere Fixierbereich (41).

Im Mantelbereich (31) hat das Gehäuse (10) z.B. zwei einander gegenüberliegende fensterartige Durchbrüche (33). Am unteren Rand des einzelnen Durchbruchs (33) ist jeweils ein Druckstab (21) als elastischer Biegebalken angeformt. Die Anformstelle für die Druckstäbe (21) liegt knapp oberhalb des Fixierbereichs (41). Zur Ausbildung des jeweiligen Druckstabs (21) befindet sich im unteren Bereich des Mantelabschnitts (31) ein schmaler, zumindest annähernd u-förmiger Spalt, der den einzelnen Druckstab (21) seitlich und oben umgibt.

Der Druckstab (21) hat beispielsweise auf 80% seiner Länge die Wandstärke und die Krümmung der Wandung des Gehäuses (10). Dieser Bereich hat unter anderem auch die Funktion eines federelastischen Biegebalkens (28). Er hat einen sichelförmigen Querschnitt.

Ggf. kann ein Teil dieses Biegebalkens (28) auch mit einem rechteckigen Querschnitt ausgestattet sein, um bei der Nutzung auftretende Biegespannungen im Biegebalkenrandbereich zu reduzieren.

Bei Injektoren, bei denen der Kolbenbetätigungsstempel (60) im Gehäuse (10) - zumindest abschnittsweise - mit geringem Spiel geradgeführt ist und der Kolbenbetätigungsstempel (60) eine ausreichende Biegefestigkeit aufweist, kann anstatt zweier oder mehrerer Druckstäbe (21) auch nur ein einziger Druckstab (21) verwendet werden.

Das hier obere freie Ende des einzelnen Druckstabs (21) wird durch den radial nach außen abstehenden Nocken (22) gebildet. Letzterer hat zumindest eine in Richtung der Mittellinie (5) orientierte Abstützfläche (23) und eine der Mittellinie (5) abgewandte Anlagefläche (24).

Im unteren Bereich des Gehäuses (10) befinden sich Halteelemente zur Befestigung der Zylinder-Kolben-Einheit (100). Die Zylinder-Kolben-Einheit (100) besteht im Ausführungsbeispiel aus einem, mit einer Injektionslösung (1) befüllbaren, transparenten Zylinder (101). In der Darstellung der Figur 1 sitzt ein Kolben (111) in der vorderen Position. Oberhalb des Kolbens (111) ist im Gehäuse (10) der Kolbenbetätigungsstempel (60) z.B. so angeordnet, dass er den Kolben (111) zwar nicht berührt, jedoch mit seinem unteren Ende z.B. im oberen Bereich des Zylinders (101) seitlich geführt wird.

Die untere Hälfte des Gehäuses (10) ist von dem hülsenartigen Auslöseelement (82) umgeben. Dieses ist z.B. im wesentlichen zylindrisch ausgebildet und beispielsweise aus Acrylnitril-Butadien-Styrol-Copolymer (ABS) hergestellt. Das Auslöseelement (82) ist auf der radialen Außenfläche (13) des Gehäuses (10) längsverschiebbar gelagert. Es endet rückwärtig mit einer scharfen Kante (85), die Teil einer stirnseitigen Rücksprungflanke (84) des Auslöseelements (82) ist. Unterhalb der Kante (85) berühren nach Figur 1 an die Druckstäbe (21) angeformte Nocken (22) mit ihren außen liegenden Anlageflächen (24) sichernd die Innenwandung (59) des Auslöseelements (82).

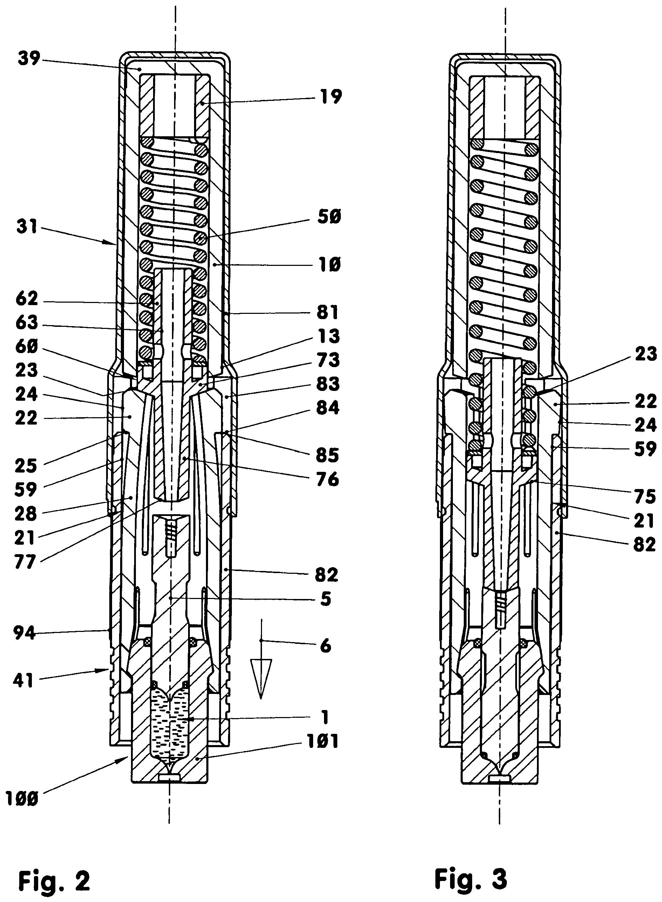

Beispielsweise in der Nähe der Kante (85) ist am Auslöseelement (82) eine Auslösekappe (81) befestigt, die das hintere Ende des Gehäuses (10) vollständig umgibt. Die Auslösekappe (81) umfasst eine umlaufende Aufweitung (83), in der beim Auslösen des Injektors die Nocken (22) aufgenommen werden, vgl. Figur 3. Anstelle dieser Aufweitung (83) können bei einem nichtrotationssymmetrischen Auslöseelement (82) pro Druckstab (21) auch partielle Aufweitungen oder nicht abgedeckte Öffnungen vorhanden sein. Oberhalb der Aufweitung (83) liegt die Auslösekappe (81) gleitfähig an der Außenwandung (13) des Gehäuses (10) an.

An das vordere Ende des Auslöseelements (82) schließt sich die Verschlusskappe (120) an. Letztere umhüllt den unteren Teil der Zylinder-Kolben-Einheit (100). Die Verschlusskappe (120) weist an der vorderen Stirnseite eine Adapteröffnung (127) mit z.B. einem Luer-Innenkegel auf, die mittels eines außenkegligen Stopfens (128) steril verschlossen ist. Die Verschlusskappe (120) ist hier auf dem unteren Bereich des Gehäuses (10) gelagert.

Der im Gehäuse (10) angeordnete Kolbenbetätigungsstempel (60) ist in zwei Bereiche aufgeteilt. Der untere Bereich ist der Kolbenschieber (76). Sein Durchmesser ist etwas kleiner als der Innendurchmesser des hinteren Bereichs des Zylinders (101). Die untere Stirnfläche des Kolbenschiebers (76) wirkt direkt auf den Kolben (111).

Der obere Bereich des Kolbenbetätigungsstempels (60), der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31). Die untere Stirnseite weist eine um den Kolbenschieber (76) herum angeordnete Bundfläche (75) auf. Sie hat die Form eines Kegelstumpfmantels, dessen Spitzenwinkel ca. 100 bis 140 Grad beträgt. Im dargestellten Ausführungsbeispiel hat die Bundfläche (75) einen Spitzenwinkel von 140 Grad. Die gedachte Spitze des Kegelstumpfmantels liegt auf der Mittellinie (5) im Bereich des Kolbenschiebers (76). Die Bundfläche (75) kann auch sphärisch gekrümmt sein.

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem oben liegenden Boden (39) des Gehäuses (10) sitzt vorgespannt die Schraubendruckfeder (50). Die Schraubendruckfeder (50) stützt sich am oben liegenden Boden (39) des Gehäuses (10) unter Zwischenschaltung einer Distanzhülse (19) ab. Die Federkraft der Schraubendruckfeder (50) wird über den Stempelteller (73) auf die Druckstäbe (21) übertragen. Aufgrund der Neigung der Bundfläche (75) werden die Druckstäbe (21) keilgetriebeartig radial nach außen gedrängt. Die Auslösehülse (82) stützt diese Radialkraft dauerhaft ab.

Der Kolbenbetätigungsstempel (60) hat oberhalb des Stempeltellers (73) einen Führungszapfen (62). Letzterer führt die Schraubendruckfeder (50) oder wird durch diese geführt. Unterhalb des Stempeltellers (73) befindet sich zentral in der Verlängerung des Führungszapfens (62) der Kolbenschieber (76), der bei einer Betätigung des Einmal-Injektors auf den Kolben (111) wirkt. Der Kolbenschieber (76) hat eine kegelmantelförmige, nach vorn gewölbte Stirnfläche (77), vgl. u.a. Figur 2. Mit dieser Stirnfläche (77) kontaktiert er beim Auslösen die komplementär geformte Stirnfläche des Kolbens (111), vgl. Figur 3. Beide Kegel haben zumindest annähernd den gleichen Kegelwinkel.

Bevor der Einweginjektor gefüllt und eingesetzt wird, ist der Federenergiespeicher (50) vorgespannt, vgl. Figur 1. Die beiden auf Druck belasteten Druckstäbe (21) halten den Kolbenbetätigungsstempel (60) an dessen Stempelteller (73) in seiner vorgespannten Lage. Dazu stützen sich die Druckstäbe (21) mit ihren Abstützflächen (23) am Stempelteller (73) ab. Die Größe der jeweiligen Kontaktfläche zwischen einer Abstützfläche (23) und der entsprechenden Stelle am Stempelteller (73) liegt im Bereich von 2 bis 20 mm². Sowohl die Abstützfläche (23) als auch die ihr zugewandte Bundfläche (75), mit der sie eine Paarung bildet, haben beispielsweise einen Mittenrauwert, der kleiner ist als zehn Mikrometer. Beispielsweise beträgt der Mittenrauwert sechs Mikrometer.

Die Biegeelastizität der Druckstäbe (21) und der Stempelteller (73) drücken die Stützstäbe (21) zumindest annähernd radial nach außen gegen das Auslöseelement (82). Dort liegen sie über Nocken (22) am Auslöseelement (82) an. Die Nocken (22) können dabei z.B. auch 5 bis 20 Millimeter unterhalb des jeweiligen freien oberen Endes der Druckstäbe (21) liegen.

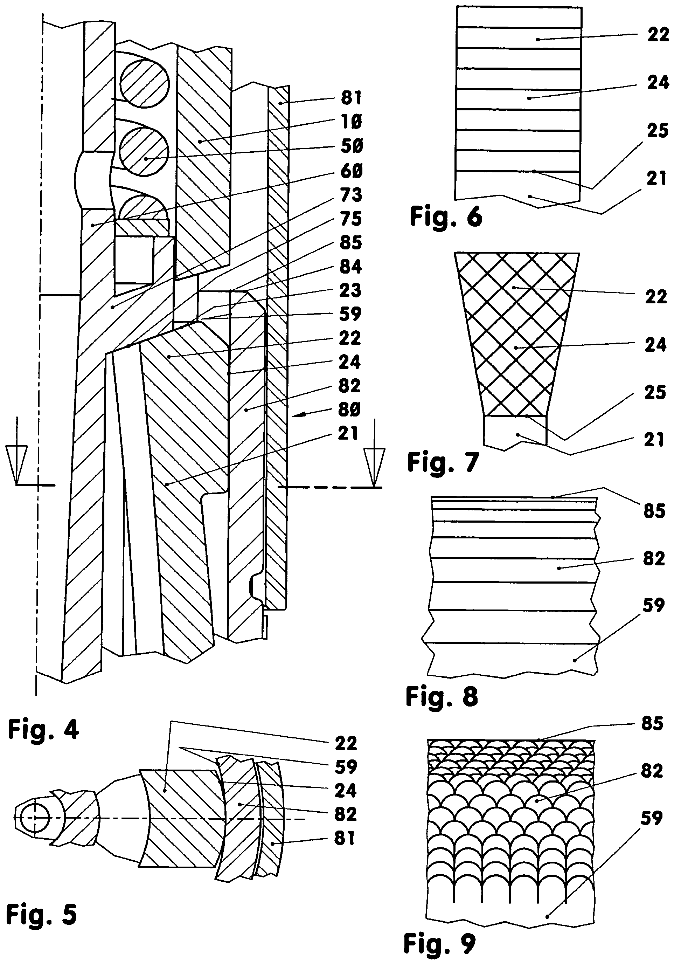

Nach den Figuren 1 und 4 liegt auf der Abstützfläche (23) der Stempelteller (73) des gespannten Einweginjektors über seine Bundfläche (75) auf. Die Abstützfläche (23), die hier die Funktion einer Keilfläche erfüllt, hat die Form eines Kegelstumpfmantels mit einem Spitzenwinkel von 140 Winkelgraden.

Ggf. haben die Druckstäbe (21) oder die Bundfläche (75) zumindest im Kontaktbereich eine keramische Panzerung. Die Bundfläche (75) kann durch eine z.B. aufgeklebte kegelstumpfmantelförmige Unterlegscheibe verstärkt sein.

Die Anlagefläche (24) der Nocken (22) ist Teil eines Konus, dessen maximaler Durchmesser z.B. 3 bis 4 Millimeter größer ist als der Außendurchmesser des Gehäuses (10). Die Anlagefläche (24) kontaktiert bei gespanntem Einweginjektor die Innenwandung (59) des hülsenartigen Auslöseelements (82). Der Kontaktbereich ist vergrößert in der Figur 4 dargestellt. Hiernach wird die Anlagefläche (24) aufgrund der Biegung des einzelnen Druckstabes (21) vor allem in ihrem unteren Bereich an die Innenwandung (59) des Auslöseelements (82) angepresst. Die Anlagefläche (24) ist strukturiert oder aufgeraut. Beispielsweise kann sie in einer Draufsicht einer Abwicklung der Anlagefläche (24) in Querrichtung angeordnete Riefen, vgl. Figur 6 oder ein Rautenmuster, vgl. Figur 7 aufweisen. Diese Muster sind beispielsweise gleichmäßig angeordnet. Der Mittenrauwert der Anlagefläche (24) ist z.B. größer als zehn Mikrometer.

In der Darstellung der Figur 7 wächst die Breite der Anlagefläche (24) des einzelnen Druckstabs (21) von unten nach oben. Hiermit ist der Anpreßdruck der Anlagefläche (24) an die Innenwandung (59) im unteren Bereich höher als im oberen. Um diesen Effekt zu verstärken, kann die Anlagefläche (24) in der Querrichtung einen kleineren Radius als die Innenwandung (59) aufweisen, vgl. Figur 5. Die Breite der Kontaktfläche kann daher kleiner sein als die Breite des einzelnen Druckstabs (21).

Um den Einweginjektor benutzen zu können, muss zunächst die Zylinder-Kolben-Einheit (100) befüllt werden. Dazu wird der Stopfen (128) aus der Adapteröffnung (127) entfernt und eine Injektionslösung (1) z.B. eingepresst oder eingesaugt. Der Kolben (111) wird hierbei zurückgedrückt oder zurückgezogen.

Zum Entsichern des Injektors wird das Sicherungselement (90), z.B. eine Abreißbanderole (94) abgezogen, so dass die Klebeverbindung zwischen der Verschlusskappe (120) und dem Auslöseelement (82) aufgehoben ist. Die Verschlusskappe (120) wird abgezogen. Der Einweginjektor wird auf der Injektionsstelle positioniert. Der Federenergiespeicher (50) ist gespannt, die Druckstäbe (21) stützen weiterhin die Bundfläche (75) des Stempeltellers (73) ab.

Nun kann das Auslöseelement (82) in Richtung der Zylinder-Kolben-Einheit (100) verschoben werden, vgl. Figur 2. Der in der Figur 2 dargestellte Zustand ist nicht statisch, er wird daher im Folgenden als fiktiver Zustand bezeichnet.

Beim Auslösen des Einweginjektors gleitet das Auslöseelement (82) auf der Außenwandung (13) des Gehäuses (10) in der Auslösebewegungsrichtung (6) linear nach unten, also in Richtung der Injektionsstelle. Die Anlageflächen (24) der Druckstäbe (21) rutschen über die Kante (85). Die Druckstäbe (21) biegen sich elastisch nach außen in ihre eigentliche Ausgangslage und springen unter der Kraft des Federelements (50) radial nach außen in die Aufweitung (83).

Die Aufweitung (83) ist im Bezug auf das Gehäuse (10) genau so positioniert und dimensioniert, dass sie die beim Auslösevorgang zurückweichenden, nach außen gedrängten Druckstäbe (21) mit ihren Nocken (22) aufnehmen kann. Die Innenkontur der Aufweitung (83) ist z.B. ein Kanal mit einer Rücksprungflanke (84), die hier eine zur Mittellinie (5) des Injektors normale Ebene darstellt. Sobald die Nocken (22) in die Aufweitung verdrängt sind, schnellt der Kolbenbetätigungsstempel (60) ungehindert nach unten, vgl. Figur 3. Der Kolben (111) wird nach unten gedrückt. Der Zylinder (100) wird entleert.

Die Innenwandung (59) des Auslöseelements (82) ist dem Bereich, an dem die Anlagefläche (24) entlang gleitet, aufgeraut oder strukturiert. Die Rauigkeitswerte entsprechen z.B. den Rauigkeitswerten der Anlagefläche (24). Bei einer Struktur kann das Muster so ausgebildet sein, wie es in den Figuren 6 oder 7 dargestellt ist.

Die Strukturdichte und/oder die Rauigkeit der Innenwandung (59) kann aber auch mit dem Hub der Relativbewegung der Anlagefläche (24) zur Innenwandung (59) des Auslöseelements (82) zunehmen. In der Figur 8 ist beispielsweise stark vergrößert ein an die scharfkantige Kante (85) angrenzendes Strukturmuster mit einander parallelen Rillen dargestellt, deren Abstand von unten nach oben abnimmt. Die Figur 9 zeigt ein Schuppenmuster, dessen Schuppen positiv und/oder negativ ausgebildet sein können. Der Abstand der Schuppen nimmt in Richtung der Relativbewegung - von unten nach oben - ab. Der unterste Bereich der Kontaktfläche der Innenwandung (59), an dem die Anlagefläche (24) vor dem Auslösen anliegt, ist beispielsweise strukturfrei.

Vor dem Auslösen und beim Auslösen des Einweginjektors drückt die Schraubenfeder (50) über den Stempelteller (73) die Nocken (22) nach außen. Aufgrund des niedrigen Gleitreibungskoeffizienten, der sich wegen der großen Kontaktfläche und der niedrigen Oberflächenrauigkeit der Paarung der Bundfläche (75) und der Abstützfläche (23) ergibt, hat dieses Keilgetriebe einen hohen Wirkungsgrad.

Um den Einweginjektor nach dem Aufsetzen auf die Injektionsstelle auszulösen, muss der Bediener zunächst die unter anderem durch die Anpresskraft verursachte Haftreibung zwischen der Anlagefläche (24) und der Innenwandung (59) überwinden. Diese Haftreibung ist umso höher, je kleiner die Kontaktfläche der Paarung und je höher die Oberflächenrauigkeit ist.

Der Anwender wird daher umso stärker den Einweginjektor gegen die Injektionsstelle drücken, je höher die Haftreibungskraft ist. Im statischen Zustand stellt damit ein hoher Rauhigkeitswert der Paarung der Anlagefläche (24) und der Innenwandung (59) sicher, dass der Anwender nach dem Aufsetzen weiterhin mit einer Mindestkraft den Einweginjektor gegen die Injektionsstelle presst.

Sobald der Anwender beim Auslösen die Haftreibungskraft überwunden hat, gleitet die Innenwandung (59) des Auslöseelements (82) entlang der Anlageflächen (24). Der Gleitreibungskoeffizient der Paarung dieser beiden Flächen (24, 59) ist abhängig von den beteiligten Werkstoffen, der Oberflächenrauigkeit und/oder der Oberflächenstruktur und der Flächenpressung. Der Gleitreibungskoeffizient dieser Paarung ist größer als der Gleitreibungskoeffizient der durch die Bundfläche (75) und die Abstützfläche (23) gebildeten Paarung. Ist beispielsweise die Anlagefläche (24) und die Innenwandung (59) mit einer Struktur nach Figur 6 ausgeführt, ist z.B. zum Verschieben des Auslöseelements (82) eine annähernd gleichbleibende hohe Vorschubkraft erforderlich. Der Anwender wird also weiterhin den Einweginjektor mit einer Kraft gegen die Injektionsstelle drücken, die gleich oder höher der für eine sichere Injektion erforderlichen Einpresskraft ist.

Der Anwender muss diese Kraft so lange aufbringen, bis die Hintergriffsflanke (25) die Kante (85) erreicht hat. Wegen der geringen Gleitreibung der durch die Bundfläche (75) und die Abstützfläche (23) gebildeten Paarung und des kleinen Keilwinkels werden die Nocken (22) schlagartig in die Aufweitungen (83) verdrängt.

Beim Auslösen eines Einweginjektors, dessen Innenwandung (59) z.B. eine Struktur nach Figur 8 aufweist, nimmt die zum Auslösen erforderliche Kraft während des Verschiebens des Auslöseelements (82) zu. Der Anwender wird so gezwungen, den Einweginjektor zunehmend stärker gegen die Injektionsstelle zu drücken. Damit ist sichergestellt, dass beim Freisetzen der Federenergie der Einweginjektor so kräftig gegen die Injektionsstelle gepresst wird, dass ein wet-shot verhindert wird.

Die erforderliche Vorschubkraft des Auslöseelements (82) zur Überwindung der Haftreibung kann geringer sein als die erforderliche Vorschubkraft zur Überwindung der Gleitreibung. Hierzu kann die Innenwandung (59) z.B. wie in der Figur 9 dargestellt ausgeführt sein. Der Bereich der Innenwandung (59), in dem die Anlagefläche (24) vor dem Auslösen anliegt, hat hier z.B. eine niedrige Oberflächenrauigkeit. Sobald die Anlagefläche (24) diesen Bereich verlassen hat, steigt die Rauigkeit und damit der Gleitreibungskoeffizient an. Beispielsweise kann der Anwender so gezwungen werden, eine gleichbleibende oder linear steigende Kraft zum Auslösen aufwenden zu müssen. Zumindest die Vorschubkraft, die unmittelbar vor dem Herausspringen der Nocken (22) aufgebracht werden muss, ist größer oder gleich der Mindestanpresskraft, die für eine sichere Injektion erforderlich ist.

Gegebenenfalls kann der Werkstoff des Kolbenbetätigungsstempels (60), der Druckstäbe (21) und des Auslöseelements (82) identisch sein.

Bei den in den Figuren dargestellten Varianten ist die einzelne Kontaktzone zwischen dem Druckstab (21) und dem Stempelteller (73), als Flächen (23) und (75) ausgeführt, die gleitfähig einander kontaktieren. In einer besonderen Ausgestaltung kann in jeder Fläche (23) der einzelnen Druckstäbe (21) eine Walze gelagert werden, die bei einer Betätigung des Injektors an der Fläche (75) des Stempeltellers wälzgelagert, also reibungsarm, abrollt.

Anstelle einer linearen Gleitbewegung des Auslöseelements (82) auf dem Gehäuse (10) kann auch eine schraubenförmige Bewegung vorgesehen werden. In diesem Fall werden das Auslöseelement (82) und das Gehäuse (10) z.B. über einen Kulissenstein und eine Kulisse aneinander geführt. Ggf. kann das Auslösen auch durch eine reine Schwenkbewegung zwischen dem Gehäuse (10) und dem Auslöseelement (82) realisiert werden. Die Schwenkachse wäre hier die Mittellinie (5).

Selbstverständlich ist es auch denkbar, die verschiedenen genannten Ausführungsformen miteinander zu kombinieren.

### Bezugszeichenliste:

- 1: Injektionslösung; Medikament
- 5: Mittellinie des Injektors, Längsrichtung
- 6: Auslösebewegungsrichtung von (82), Abwärtsbewegung Richtungspfeil

- 10: Gehäuse, einteilig
- 13: Außenfläche, zylindrisch
- 19: Distanzhülse

- 21: Druckstäbe, Stützstäbe; Zughaken
- 22: Nocken
- 23: Abstützfläche
- 24: Anlagefläche
- 25: Hintergriffsflanke
- 28: Biegebalken

- 31: Mantelbereich
- 33: Durchbrüche
- 39: Boden

- 41: Fixierbereich für die Zylinder-Kolben-Einheit

- 50: Federelement, Schraubendruckfeder, Federenergiespeicher
- 59: Innenwandung von (82)

- 60: Kolbenbetätigungsstempel
- 62: Führungszapfen
- 63: Bohrung, Durchgangsbohrung

- 73: Stempelteller
- 75: Bundfläche, konisch
- 76: Kolbenschieber
- 77: Kolbenstirnfläche, kegelmantelförmig

- 80: Auslöseeinheit
- 81: Auslösekappe
- 82: Auslöseelement
- 83: Aufweitung
- 84: Rücksprungflanke
- 85: Kante, scharfkantig

- 90: Originalitätsverschluß, Banderole, Sicherungselement
- 94: Abreißbanderole

- 100: Zylinder-Kolben-Einheit
- 101: Zylinder

- 111: Kolben

- 120: Verschlusskappe, Klebeversiegelung
- 127: Adapteröffnung
- 128: Stopfen

## Patentansprüche

1. Nadelloser Einweginjektor mit
- einem Gehäuse (10), das mindestens einen Druckstab (21) umfasst,
- mit einer Zylinder-Kolben-Einheit (100),
- mit einem einen Stempelteller (73) aufweisenden Kolbenbetätigungsstempel (60),
- mit einer ein hülsenartiges Auslöseelement (82) aufweisenden Auslöseeinheit (80) und
- mit einem zwischen einem Boden (39) des Gehäuses (10) und dem Stempelteller (73) vorgespannt sitzenden Federenergiespeicher (50),
- wobei beim gespannten Einweginjektor der Stempelteller (73) über eine Bundfläche (75) auf einer Abstützfläche (23) des Druckstabs (21) aufliegt,
- wobei eine Anlagefläche (24) eines Nockens (22) des Druckstabs (21) eine Innenwandung (59) des Auslöseelements (82) kontaktiert,
- wobei das Auslöseelement (82) eine scharfe Kante (85) aufweist,
- wobei die Auslöseeinheit (80) den vorgespannten Federenergiespeicher (50) zunächst mittels des Druckstabs (21) verriegelt,
- wobei das Auslöseelement (82) in Richtung der Zylinder-Kolben-Einheit (100) verschiebbar ist,
- wobei beim Auslösen der Druckstab (21) am Auslöseelement (82) entlang gleitet ist und die Anlagefläche (24) über die Kante (85) rutscht und
- wobei nach dem Auslösen der Auslöseeinheit (80) der Federenergiespeicher (50) mittels des Kolbenbetätigungsstempels (60) den Druckstab (21) verdrängt und die Zylinder-Kolben-Einheit (100) betätigt, **dadurch gekennzeichnet,**
- **dass** bei Belastung durch den vorgespannten Federenergiespeicher (50) die Paarung der einander zugewandten Innenwandung (59) des Auslöseelements (82) und der Anlagefläche (24) des Druckstabs (21) zumindest bereichsweise einen höheren Gleitreibungskoeffizienten aufweist als die Paarung der einander zugewandten Bundfläche (75) des Kolbenbetätigungsstempels (60) und der Abstützfläche (23) des Druckstabs (21), wobei die Innenwandung (59) in dem Bereich, an dem die Anlagefläche (24) entlang gleitet, zur Erhöhung des Gleitreibungskoeffizienten aufgeraut oder strukturiert ist.

2. Einweginjektor nach Anspruch 1,
**dadurch gekennzeichnet,**
**dass** das Auslöseelement (82) hülsenförmig zumindest bereichsweise das Gehäuse (10) umgreift.

3. Einweginjektor nach Anspruch 1,
**dadurch gekennzeichnet,**
**dass** der Einweginjektor zwei Druckstäbe (21) umfasst.

4. Einweginjektor nach Anspruch 1,
**dadurch gekennzeichnet,**
**dass** der Mittenrauwert der Anlagefläche (24) und/oder der Innenwandung (59) zumindest im Kontaktbereich dieser Flächen (24, 59) größer oder gleich zehn Mikrometer ist.

5. Einweginjektor nach Anspruch 1,
**dadurch gekennzeichnet,**
**dass** die Rauigkeit der Innenwandung des Auslöseelements (82) in Richtung der scharfkantigen Kante (85) zunimmt.

6. Einweginjektor nach Anspruch 1,
**dadurch gekennzeichnet,**
**dass** die Breite der genutzten Anlagefläche (24) kleiner ist als die Breite des einzelnen Druckstabs (21).

## Claims

1. A needle-less single-use injector, having
- a housing (10) which comprises at least one press rod (21),
- having a cylinder/piston unit (100),
- having a piston actuating plunger (60) comprising a plunger plate (73),
- having a triggering unit (80) comprising a sleeve-like triggering element (82), and
- having a spring energy store (50) which is positioned and which is pre-tensioned between a floor (39) of the housing (10) and the plunger plate (73),
- wherein, when the single-use injector is tensioned, the plunger plate (73) rests on a support surface (23) of the press rod (21) via its collar surface (75),
- wherein an abutment surface (24) of a cam (22) of the press rod (21) contacts an inner wall (59) of the triggering element (82),
- wherein the triggering element (82) comprises a sharp edge (85),
- wherein the triggering unit (80) initially locks the pre-tensioned spring energy store (50) by means of the press rod (21),
- wherein the triggering element (82) is displaceable in the direction of the cylinder/piston unit (100),
- wherein, on triggering, the press rod (21) slides along the triggering element (82) and the abutment surface (24) slips over the edge (85), and
- wherein, after triggering of the triggering unit (80), the spring energy store (50) displaces the press rod (21) by means of the piston actuating plunger (60) and actuates the cylinder/piston unit (100),
**characterized in that**
when loaded by the pre-tensioned spring energy store (50), the pairing of the mutually facing inner wall (59) of the triggering element (82) and the abutment surface (24) of the press rod (21) comprises, at least in certain regions,: a higher coefficient of sliding friction than the pairing of the mutually facing collar surface (75) of the piston actuating plunger (60) and of the support surface (23) of the press rod (21) wherein, in the region on which the abutment surface (24) slides, the inner wall (59) is roughened or structured in order to increase the coefficient of sliding friction.

2. The single-use injector as claimed in claim 1,
**characterized in that**
the triggering element (82) surrounds the housing (10) in a sleeve-like manner, at least in certain regions.

3. The single-use injector as claimed in claim 1,
**characterized in that**
the single-use injector comprises two press rods (21).

4. The single-use injector as claimed in claim 1,
**characterized in that**
the abutment surface (24) and/or the inner wall (59) have a mean roughness value greater than or equal to ten micrometres, at least in the region of contact of said surfaces (24, 59).

5. The single-use injector as claimed in claim 1,
**characterized in that**
the roughness of the inner wall of the triggering element (82) increases in the direction of the sharp-edged edge (85).

6. The single-use injector as claimed in claim 1,
**characterized in that**
the width of the abutment surface (24) which is used is smaller than the width of the individual press rod (21).

## Revendications

1. Injecteur sans aiguille à usage unique composé
- d'un boîtier (10) comprenant au moins une tige de pression (21),
- d'une unité cylindre-piston (100),
- d'un poinçon d'actionnement de piston (60) présentant une plaque d'appui (73),
- d'une unité de déclenchement (80) équipée d'un élément de déclenchement (82) en forme de manchon et
- d'un accumulateur d'énergie à ressort (50) précontraint placé entre un fond (39) du boîtier (10) et la plaque d'appui (73),
- la plaque d'appui (73), lorsque l'injecteur à usage unique est sous contrainte, reposant sur une surface d'appui (23) de la tige de pression (21) au moyen d'une surface d'épaulement (75),
- une surface de contact (24) d'une came (22) de la tige de pression (21) contactant une paroi intérieure (59) de l'élément de déclenchement (82),
- l'élément de déclenchement (82) présentant une arête vive (85),
- l'unité de déclenchement (80) verrouillant l'accumulateur d'énergie à ressort (50) précontraint d'abord au moyen de la tige de pression (21)
- l'élément de déclenchement (82) pouvant être déplacé en direction de l'unité cylindre-piston (100).
- lors du déclenchement la tige de pression (21) glissant le long de l'élément de déclenchement (82) et la surface de contact glissant par-dessus l'arête (85) et
- après le déclenchement de l'unité de déclenchement (80) l'accumulateur d'énergie à ressort (50) déplaçant la tige de pression (21) au moyen du poinçon d'actionnement du piston (60) et actionnant l'unité cylindre-piston (100),
**caractérisé en ce**
**que**, lors de l'application d'une contrainte par l'intermédiaire de l'accumulateur d'énergie à ressort précontraint (50), la paire formée par les parois intérieures (59) de l'élément de déclenchement (82) et, face à celle-ci, de la surface de contact (24) de la tige de pression (21) présente, au moins par endroits, un coefficient de frottement dynamique supérieure à celui de la paire formée par la surface d'épaulement (75) du poinçon d'actionnement du piston (60) et de la surface d'appui (23) de la tige de pression (21), la paroi intérieure (59) dans la zone où la surface de contact coulisse le long de la paroi, étant rugueuse ou structurée afin d'augmenter le coefficient de frottement dynamique.

2. Injecteur à usage unique selon la revendication 1,
**caractérisé en ce**
**que** l'élément de déclenchement (82) réalisé en forme de manchon entoure le boîtier (10) au moins par endroits.

3. Injecteur à usage unique selon la revendication 1,
**caractérisé en ce**
**que** l'injecteur à usage unique comprend deux barres de pression (21).

4. Injecteur à usage unique selon la revendication 1,
**caractérisé en ce**
**que** la rugosité moyenne de la surface de contact (24) et/ou de la paroi intérieure (59) est supérieure ou égale à 10 micromètres tout au moins dans la zone de contact de ces deux surfaces (24, 59).

5. Injecteur à usage unique selon la revendication 1,
**caractérisé en ce**
**que** la rugosité de la paroi intérieure de l'élément de déclenchement (82) augmente dans la direction de l'arête vive (85).

6. Injecteur à usage unique selon la revendication 1,
**caractérisé en ce**
**que** la largeur de la surface de contact utilisée (24) est inférieure à la largeur de chaque barre de pression (21).
